# EUROPEAN PATENT SPECIFICATION

(11) **EP 2 201 936 B1**
(45) Date of publication and mention of the grant of the patent: **19.06.2013**
(21) Application number: 10003076.6
(22) Date of filing: 19.01.2005
(51) Int. Cl.: A61K 9/12, A61K 31/58, A61P 11/00, A61K 9/00

(54) **Method of treating acute rhinosinusitis**
Verfahren zu Behandlung akuter Rhinosinusitis
Procédé de traitement de la rhinosinusite aiguë

(30) Priority: 21.01.2004 US 537830 P
(43) Date of publication of application: 30.06.2010
(62) Divisional of application: 05711867.1
(73) Proprietor: Merck Sharp & Dohme Corp., Rahway, NJ 07065-0907 (US)
(72) Inventor: Bloom, Melvyn, Chatam, NJ 07938 (US); Danzig, Melvyn, Morganville, NJ 07751 (US); Rohane, Patricia, Randolph, NJ 07869 (US); Staudinger, Heribert W., Green Brook, NJ 08812 (US)
(74) Representative: Horgan, James Michael Frederic

(56) References cited:
- EP-A- 1 174 138
- WO-A-00/27373
- NAYAK A S ET AL: "EFFECTIVE DOSE RANGE OF MOMETASONE FUROATE NASAL SPRAY IN THE TREATMENT OF ACUTE RHINOSINUSITIS" ANNALS OF ALLERGY, ASTHMA & IMMUNOLOGY, ARLINGTON HEIGHTS, IL, US, vol. 89, no. 3, September 2002 (2002-09), pages 271-278, XP008058401 ISSN: 1081-1206
- PUHAKKA T ET AL: "SINUSITIS IN THE COMMON COLD" JOURNAL OF ALLERGY AND CLINICAL IMMUNOLOGY, MOSBY - YEARLY BOOK, INC, US, vol. 102, no. 3, September 1998 (1998-09), pages 403-408, XP001079208 ISSN: 0091-6749
- PARIKH A ET AL: "TOPICAL CORTICOSTEROIDS IN CHRONIC RHINOSINUSITIS: A RANDOMIZED, DOUBLE-BLIND, PLACEBO-CONTROLLED TRIAL USING FLUTICASONE PROPIONATE AQUEOUS NASAL SPRAY" RHINOLOGY, LEIDEN, NL, vol. 39, no. 2, June 2001 (2001-06), pages 75-79, XP008058638 ISSN: 0300-0729

## Description

### BACKGROUND OF THE INVENTION

Virtually all persons are occasionally stricken with acute upper respiratory infections, acute or chronic allergy flare-ups of the nose, and/or acute or chronic non-allergic rhinosinusitis. Significant discomfort and inconvenience are usually incurred by persons afflicted by such conditions. All of these disorders are characterized by intense inflammation of the nasal membranes. A number of symptoms which, at least in part, contribute to the discomfort and inconvenience associated with the common cold or other rhinosinusitis symptoms often include one or more of the following: nasal congestion, post-nasal drip, decreased sense of smell, ear fullness, headache, sore throat, malaise, muscle and joint aches, fatigue, cough, chest congestion, fever, chills and gastrointestinal maladies. Considerable research has been conducted over the years aimed at reducing the incidence and duration of symptoms associated with allergies and common colds, and at suppressing or eliminating their accompanying symptoms.

The most common cause for acute rhinosinusitis is a viral cold or flu that infects the upper respiratory tract and causes obstruction. This obstruction often times creates an environment that is hospitable for bacteria. Bacterial rhinosinusitis usually follows a viral infection or allergic rhinitis. Antibacterial therapy is the standard treatment for acute rhinosinusitis, despite the fact that the inflammation is usually caused by virayl pathogens. Amoxicillin or other antibiotics are customarily used as first-line therapy for acute rhinosinusitis.

Adjunct therapy for acute community acquired rhinosinusitis includes oral decongestants, cough suppressants, antihistamines and steroidal and non-steroidal anti-inflammatory agents delivered orally or topically. In one study, Mometasone Furoate Nasal Spray, as adjunctive treatment with an oral antibiotic, significantly improved the symptoms of rhinosinusitis. Nayak, et al., Ann. All. Asthma Immunol. 2002 Sep; 89(3): 271-8; See also Charous et al., J. All. Clin. Immunology, (105) S210 (2000).

Due to the complex and diverse causes of acute rhinosinusitis, there exists a need for new and improved methods of treating this disease.

### SUMMARY OF THE INVENTION

Accordingly, the present invention provides a composition for use in treating acute rhinosinusitis of the upper airway passages in patients afflicted with said disease without the concomitant administration of an antibiotic, wherein the composition is to be administered twice a day to the surfaces of said passages of said patients an amount of aerosolized particles of a mometasone furoate effective for treating said disease.

The corticosteroid in the composition can be formulated for administering in the form of an aqueous suspension. Alternatively, the corticosteroid in the composition can be formulated for administering in the form of a dry powder. In one embodiment, the the mometasone furoate in the composition is mometasone furoate monohydrate. In a further embodiment, the composition is an aqueous suspension of mometasone furoate monohydrate and the amount to be administered is 200µg.

### DETAILED DESCRIPTION OF THE INVENTION

Mometasone furoate is a corticosteroid approved for topical dermatologic use to treat inflammatory and/or pruritic manifestations of corticosteroid-responsive dermatoses. The compound may be prepared in accordance with the procedures disclosed in U.S. Patent Nos. 4,472,393, 4,731,447, 4,873,335, 5,837,699 and 6,127,353. Mometasone is a topically active steroid which is not readily bioavailable would provide a therapeutic advantage over other topically active corticosteroids that are more systematically bioavailable and it would also be superior to any corticosteroid orally administered by the oral swallowing of, for example, a solution, tablet or capsule. It is commercially available as a spray for intra-nasal administration under the name of Nasonex®. Mometasone's use for the treatment of airway passages and lung diseases is disclosed in U.S. Patent Nos. 6,677,323, 6,677,322, 6,365,581, 6,187,765, 6,068,832, 6,057,307 5,889,015 5,837,699 and 5,474,759.

Although corticosteroids have been effective in treating airway passage diseases such as asthma, such treating with corticosteroids may often cause systemic side-effects such as suppression of hypothalamic-pituitary-adrenocortical ("HPA") axis function by reducing corticot (ACTH) production, which in turn leads to a reduced cortisol secretion by the adrenal gland.

Mometasone furoate exhibits superior anti-inflammatory effects in treating airway passage diseases such as allergic rhinitis by acting on surfaces of the upper airway passages while having a substantially minimum systemic effect. The substantial minimization of the systemic effect of mometasone furoate administered intranasally has been measured by High Performance Liquid Chromatography (HPLC) metabolite profiling of plasma radioactivity of mometasone furoate, its substantially complete (>98%) first-pass metabolism in the liver and by a minimal reduction in cortisol secretion levels.

The term "therapeutic index", as used herein, means the ratio of local efficacy to systemic safety. Systemic safety of such cortisteroids is usually measured by HPA-axis function; other measures of systemic effect include, for example, growth suppression, bone density, and skin thickness measurements.

In addition to the superb safety profile exhibited by mometasone furoate administered to patients with acute rhinosinusitis in accordance with the present invention, mometasone furoate also exhibits an unexpected higher level of efficacy in treating acute rhinosinusitis rhinitis than the superb safety profile would suggest.

The devices found useful for providing measured substantially non-systematically bioavailable amounts of aerosolized mometasone furoate or aerosolized pharmaceutical compositions thereof for delivery to the oral airway passages and lungs by oral inhalation or intranasally by inhalation include pressurized metered-dose inhalers ("MDI") which deliver aerosolized particles suspended in chlorofluorocarbon propellants such as CFC-11, CFC-12, or the non-chlorofluorocarbons or alternate propellants such as the fluorocarbons, HFC-134A or HFC-227 with or without surfactants and suitable bridging agents; dry-powder inhalers either breath activated or delivered by air or gas pressure such as the dry-powder inhaler disclosed in the Schering Corporation International Patent Application No. PCT/US92/05225, published 7 Jan., 1993 as well as the TURBUHALER® (available from Astra Pharmaceutical Products, Inc.) or the ROTAHALER® (available from Allen & Hanburys) which may be used to deliver the aerosolized mometasone furoate as a finely milled powder in large aggregates either alone or in combination with some pharmaceutically acceptable carrier e.g. lactose; and nebulizers. The inhalation of aerosolized drugs by use of nebulizers and metered-dose inhalers such as used to deliver Nasonex® inhalation aerosol (available from Schering Corporation, Kenilworth, N.J.) is disclosed in Remington's Pharmaceutical Sciences, Mack Publishing Co. Easton, Pa., 15th Ed. Chapter 99, pages 1910-1912.

Mometasone furoate may be also administered in specific, measured amounts in the form of an aqueous suspension by use of a pump spray bottle such as the bottles used to deliver NASONEX® Nasal Spray as well as the spray bottle disclosed in the Schering Corporation Industrial Design Deposit DM/026304, registered by the Hague Union on Jun. 1, 1993 (each are available from Schering Corporation). The aqueous suspension compositions of the present invention may be prepared by admixing mometasone furoate (which may be in the form of mometasone furoate monohydrate) with water and other pharmaceutically acceptable excipients. See International Application No. PCT/US91/06249 especially Examples 1-5 for preparation of mometasone furoate monohydrate and aqueous suspensions containing same.

The aqueous suspensions of the invention may contain from about 0.01 to 10.0 mg, preferably 0.1 to 10.0 mg of mometasone furoate monohydrate per gram of suspension. The aqueous suspension compositions according to the present invention may contain, inter alia, water, auxiliaries and/or one or more of the excipients, such as: suspending agents, e.g., microcrystalline cellulose, sodium carboxymethylcellulose, hydroxpropyl-methyl cellulose; humectants, e.g. glycerin and propylene glycol; acids, bases or buffer substances for adjusting the pH, e.g., citric acid, sodium citrate, phosphoric acid, sodium phospate as well as mixtures of citrate and phosphate buffers; surfactants, e.g. Polysorbate 80; and antimicrobial preservatives, e.g., benzalkonium chloride, phenylethyl alcohol and potassium sorbate. The spray may be scented or unscented.

Based on the judgment of the attending clinician, the amount of mometasone furoate administered and the treatment regimen used will, of course, be dependent on the age, sex and medical history of the patient being treated, the severity of the specific condition and the tolerance of patient to the treatment regimen as evidenced by local toxicity (e.g., nasal irritation and/or bleeding) and by systemic side-effects (e.g. cortisol level). Cortisol (also referred to as hydrocortisone) is the major natural glucocorticosteroid elaborated by the adrenal cortex.

For the treatment of acute rhinosinusitis, the substantially non-systematically bioavailable amount of mometasone furoate which may be administered as an aqueous suspension or dry powder is in the range of about 10 to 5000 µg ("µg")/day, 10 to 4000 µg/day, 10 to 2000 µg/day, 25-1000 µg/day, 25 to 400 µg/day, 25-200 µg/day, 25-100 µg/day or 25-50 µg/day in single or divided doses.

In treating allergic and non-allergic rhinitis, the aqueous suspension of mometasone furoate may be administered intranasally by inserting an appropriate device (such as the pump spray bottle used to deliver NASONEX AQ® Nasal Spray as well as the spray bottle disclosed in the Schering Corporation Industrial Design Deposit DM/026304 registered Jun. 1, 1993) into each nostril. Active drug is then expelled (nasal spray device) or could be nasally inhaled (sniffed) as a powder. Efficacy is generally assessed in a double blind fashion by a reduction in nasal symptoms (e.g., sneezing, itching, congestion, and discharge). Other objective measurements (e.g., nasal peak flow and resistance) can be used as supportive indices of efficacy.

The following dosage ranges of mometasone furoate may be used: (1) for metered dose inhalers with standard CFC or alternate propellant about 10 to 5000 µg/day or 10 to 4000 µg/day or 10 to 2000 µg/day, or 50 to 1000 µg/day or 25 to 100 µg/day, or 25 to 400 µg/day, or 25 to 200 µg/day, or 25-50 µg/day; (2) for the dry powder inhaler--about 10 to 5000 µg/day or 10-4000 µg/day or 10-2000 µg/day or 25-1000 µg/day or 25-400 µg/day or 25-200 µg/day or 50-200 µg/day or 25-50 µg/day of anhydrous mometasone furoate; ; typically the metered dose inhaler unit will contain 120 doses; (3) for aqueous suspension for inhalation, the preferral dosage ranged from 25 to 800 µg/100 µL and the dosages are 25, 50, 100, 125, 150, 175, 200, 225, 250, 300, 400, 500 and 800 µg/100 µL of mometasone furoate divided doses. The aqueous suspension of mometasone furoate has been found to be safe and effective in treating allergic rhinitis e.g. seasonal allergic rhinitis from 25 µg up to 1600 µg administered once-a-day; the preferred dosage range is 25-800 µg a day, although no improvement in treatment is typically found above 400 µg a day. The most preferred dosages are 25, 50 and 100 µg administered twice to each nostril, once-a-day for a total once-a-day dose of 100, 200 and 400 µg. Typically 2-4 suspension of mometasone furoate monohydrate may be placed in a plastic nebulizer container and the patient would inhale for 2-10 minutes. The total dosage placed in such a container would be in the range of 300-3000 µg.

In a preferred aspect of this invention, the anhydrous mometasone furoate may be admixed with a dry excipient, for example dry lactose for use in the dry powder inhaler. The mometasone furoate:dry lactose ratio varies broadly from 1:19 to 1:0, and preferably it is 1:19 to 1:4. Typically, the suitable anhydrous mometasone furoate dosage range is 25 to 600 µg administered once-a-day. The preferred mometasone furoate dosages for admixture with dry lactose are 25, 100, 200 and 250 µg which are administered in one to three puffs a day. The preferred combined mometasone furoate:lactose dose is 500 µg for each dose. For example, for the preferred 1:19 ratio, 25 µg of anhydrous mometasone furoate are admixed with 475 µg of anhydrous lactose and for the preferred 1:4 ratio, 100 µg of anhydrous mometasone furoate are admixed with 400 µg of anhydrous lactose, to produce the 500 µg dose of the mometasone furoate:lactose admixture.

For any route of administration, divided doses may be used. For example, when a metered dose inhaler is used to deliver.

When a nebulizer container is used to deliver for example 200 µg a day of an aqueous suspension of mometasone furoate, two squeezes of 50 µg into each nostril would normally be used to deliver the drug.

The invention will be further described with regards to the following example.

### Example 1

A Phase-2, dose-ranging, double-blind, double-dummy, randomized, multi-center, multi-national study with 4 parallel groups was carried out. There were three treatment regimens: Mometasone Furoate Nasal Spray 200 µg was administered once a day; Mometasone Furoate Nasal Spray 200 µg also was administered twice a day; Amoxicillin 500 mg was administered three times a day. All three were administered against placebo. The regimens were administered to subjects 12 years of age or older with acute rhinosinusitis that was clinically diagnosed with symptoms present for 7 days or more and 28 days or less. Subjects with a major symptom score (sum of symptoms - facial pain, rhinorrhea, post nasal drip, sinus headache, nasal congestion- each scored 0 - none- to 3 -severe) greater than or equal to 5 and less than or equal to 12 at baseline with less than or equal to 3 symptoms scored severe were included. Subjects with a bacterial rhinosinusitis suspected on the presence of fever greater than or equal to 101°F/38.3°C, persistent severe unilateral facial pain/tooth pain, orbital or peri-orbital facial swelling, dental involvement; and/or worsening symptoms after initial improvement were excluded from the study.

The primary efficacy variable of the study was the actual major symptom score (averaged daily for AM and PM assessments over 15 days of treatment). Subjects were followed for an additional 14 days to assess possible recurrence.

The following results were obtained.

| **Number of subjects** | **Mometasone Furoate Nasal Spray 200 µg once a day** | **Mometasone Furoate Nasal Spray 200 µg twice a day** | **Amoxicillin** | **Placebo** | **Total** |
|---|---|---|---|---|---|
| Treatment phase | 243 | 235 | 251 | 252 | 981 |
| Follow-up phase | 235 | 230 | 245 | 241 | 951 |

65% of subjects were female, 42% Caucasian, aged 12 to 76 years. For the major symptom score, Mometasone Furoate Nasal Spray 200 µg twice a day showed superior efficacy vs. placebo (p< 0.001) and vs. Amoxicillin (p=0.002). Mornetasone Furoate Nasal Spray 200 µg once a day showed superior efficacy vs. Placebo (p=0.018), but not vs. Amoxicillin. Amoxicillin was not different from placebo (p=0.275). Mometasone Furoate Nasal Spray 200 µg twice a day demonstrated improvement in symptom scores vs. placebo beginning at Day 2 (p<=0.037) and vs. Amoxicillin beginning at Day 4 (p<=0.012). The treatment failed in more subjects treated with placebo (n=27) than with Mometasone Furoate Nasal Spray 200 µg twice a day (n=11) (p=0.017). Of 951 subjects who entered the follow-up phase, 63 subjects experienced recurrence of symptoms with no significant difference among treatment groups. The recurrence rate was almost identical between Mometasone Furoate Nasal Spray 200 µg twice a day, placebo and Amoxicillin (7, 7 and 8 %, respectively) The therapeutic response at endpoint was better with Mometasone Furoate Nasal Spray 200 µg twice a day than with placebo (p=0.001) or with Amoxicillin (p=0.013).

155 of the subjects (15.8%) experienced treatment related adverse events mostly mild to moderate in severity with no difference among groups. Epistaxis was the most common adverse event (3.2%-5.1%) followed by headache (1.2%-3.6%).

In conclusion, Mometasone Furoate Nasal Spray 200 µg twice a day demonstrated superior efficacy vs. placebo and Amoxicillin in the treatment of clinically diagnosed community acquired acute rhinosinusitis while amoxicillin ways not different from placebo. Mometasone Furoate Nasal Spray 200 µg twice a day demonstrated improvements in symptom relief vs. placebo and Amoxicillin, as early as at Day 2 and Day 4, respectively. During the follow-up phase, the recurrence rates were similar between the treatment groups. Reported adverse events were mild to moderate, low in incidence, and with similar rates between the groups.

It will be appreciated by one of skill in the art that other corticosteroids may be used within the scope of the present invention. Other corticosteroids for use in the present invention, without limitation, include butoxicart, rofleponide, budesonide, deflazacort, ciclesonide, fluticasone, beclomethasone, loteprednol or triamcinolone. For instance, when the corticosteroid is fluticasone, it may be administered at the dose of 2 sprays of 50 µg of fluticasone propionate each in each nostril once daily. Alternatively, it may be administered at a dose of fluticasone is 1 spray of 50 µg of fluticasone propionate each in each nostril once daily. When the corticosteroid is triamcinolone, it may be administered at a dose of triamcinolone is 220 µg per day as two sprays in each nostril once daily. Alternatively, it may be administered at a dose of 110 µg per day as one spray in each nostril once daily. When the corticosteroid is budesonide, the administered dose of budesonide may be 64 µg per day administered as one spray per nostril of 32 µg once daily.

## Claims

1. A composition for use in treating acute rhinosinusitis of the upper airway passages in patients afflicted with said disease without the concomitant administration of an antibiotic, wherein the composition is to be administered twice a day to the surfaces of said passages of said patients an amount of aerosolized particles of a mometasone furoate effective for treating said disease.

2. The composition for use in treating acute rhinosinusitis as in claim 1, wherein the corticosteroid in the composition is formulated for administering in the form of an aqueous suspension.

3. The composition for use in treating acute rhinosinusitis as in claim 1, wherein the corticosteroid in the composition is formulated for administering in the form of a dry powder.

4. The composition for use in treating acute rhinosinusitis as in claim 1, wherein the mometasone furoate is mometasone furoate monohydrate.

5. The composition for use in treating acute rhinosinusitis as in claim 1, wherein the composition is an aqueous suspension of mometasone furoate monohydrate and the amount to be administered is 200µg.

## Patentansprüche

1. Eine Zusammensetzung zur Verwendung bei der Behandlung von akuter Rhinosinusitis der oberen Atemwege bei Patienten, die an dieser Erkrankung leiden, ohne die gleichzeitige Verabreichung eines Antibiotikums, wobei die Zusammensetzung zweimal am Tag als aerosolisierte Mometasonfuroat-Teilchen in einer Menge, die eine Behandlung der Erkrankung bewirkt, auf die Oberflächen der Atemwege der Patienten zu verabreichen ist.

2. Die Zusammensetzung zur Verwendung zur Behandlung von akuter Rhinosinusitis wie in Anspruch 1, wobei das Kortikosteroid in der Zusammensetzung zur Verabreichung in Form einer wässrigen Suspension formuliert ist.

3. Die Zusammensetzung zur Verwendung zur Behandlung von akuter Rhinosinusitis wie in Anspruch 1, wobei das Kortikosteroid in der Zusammensetzung zur Verabreichung in Form eines Trockenpulvers formuliert ist.

4. Die Zusammensetzung zur Verwendung zur Behandlung von akuter Rhinosinusitis wie in Anspruch 1, wobei das Mometasonfuroat Mometasonfuroat-Monohydrat ist.

5. Die Zusammensetzung zur Verwendung zur Behandlung von akuter Rhinosinusitis wie in Anspruch 1, wobei die Zusammensetzung eine wässrige Suspension von Mometasonfuroat-Monohydrat ist und die zu verabreichende Menge 200 µg beträgt.

## Revendications

1. Composition à utiliser dans le traitement de la rhinosinusite aiguë des passages des voies aériennes supérieures chez des patients souffrant de ladite maladie sans l'administration concomitante d'un antibiotique, où la composition doit être administrée deux fois par jour aux surfaces desdits passages desdits patients en une quantité de particules en aérosol d'un furoate de mométasone efficace pour traiter ladite maladie.

2. Composition à utiliser dans le traitement de la rhinosinusite aiguë selon la revendication 1, dans laquelle le corticostéroïde dans la composition est formulé pour administration sous forme d'une suspension aqueuse.

3. Composition à utiliser dans le traitement de la rhinosinusite aiguë selon la revendication 1, dans laquelle le corticostéroïde dans la composition est formulé pour administration sous forme d'une poudre sèche.

4. Composition à utiliser dans le traitement de la rhinosinusite aiguë selon la revendication 1, dans laquelle le furoate de mométasone est du furoate de mométasone monohydraté.

5. Composition à utiliser dans le traitement de la rhinosinusite aiguë selon la revendication 1, où la composition est une suspension aqueuse de furoate de mométasone monohydraté et la quantité à administrer est de 200 µg.
